# EUROPEAN PATENT APPLICATION

(11) **EP 4 768 065 A1**
(43) Date of publication of application: **01.07.2026**
(21) Application number: 24952278.0
(22) Date of filing: 27.08.2024
(51) Int. Cl.: A61M 25/10

(54) **LIQUID CORONA DISCHARGE-BASED BALLOON CATHETER AND CONTROL METHOD**

(30) Priority: 26.08.2024 CN 202411175904
(71) Applicant: Cyber-VP Medical Device (Shenzhen) Co., Ltd., Shenzhen, Guangdong 518122 (CN)
(72) Inventor: OUYANG, Junxiong, Shenzhen, Guangdong 518122 (CN); LU, Lizhong, Shenzhen, Guangdong 518122 (CN)
(74) Representative: Tahtadjiev, Konstantin
(86) International application number: PCT/CN2024/114942
(87) International publication number: WO 2026/044519

(57) **Abstract**

The present invention provides a balloon catheter based on liquid corona discharge and its control method, the balloon catheter comprises an inner tube; a balloon closedly arranged around the inner tube; a corona generator located in the containment space between the inner tube and the balloon where electrolyte liquid is stored; a power pulse generator electrically connected to the corona generator; the corona generator is located in the electrolyte liquid in the containment space between the balloon and the inner tube; the corona generator receives the pulse electrical signals from the power pulse generator, the electrolyte liquid undergoes a corona reaction and generates molecular ionization, and the vapor bubbles produced by molecular ionization compress the electrolyte liquid, increasing the pressure within the balloon and driving the balloon to expand radially along the inner tube, thereby improving the safety, ease of operation, reusability, and precision control of the balloon catheter.

## Description

### BACKGROUND OF THE PRESENT INVENTION

### FIELD OF INVENTION

The present invention relates to the field of balloon catheters, and particularly relates to a balloon catheter based on liquid corona discharge and its control method.

### DESCRIPTION OF RELATED ARTS

In the field of medical devices, balloon catheters are mainly used to dilate narrowed blood vessels, and this treatment method has been proven to be safe and effective. Currently, balloon catheters primarily use the principle of arc discharge for balloon expansion. Arc discharge is a phenomenon where a high-density plasma channel is formed through a medium under high voltage. In arc discharge, the medium between the electrodes is completely broken down under high voltage conditions, forming a continuous high-temperature, high-density plasma channel. Arc discharge is a strong discharge phenomenon characterized by high temperatures and high current density. Arc discharge is a holistic effect, with the arc penetrating the medium between the electrodes and forming a continuous high-temperature, high-current channel. Under a sufficiently high electric field, the liquid medium is completely broken down, forming a high-density plasma channel. This process is accompanied by intense energy release and high-temperature generation. The shock wave energy of arc discharge is enormous and difficult to control precisely, potentially causing unpredictable damage to surrounding tissues. The shock wave of arc discharge is strong, although the arc discharge's shock wave can quickly break up calcified plaques, the strong impact on the surrounding blood vessels may cause vascular injury or other complications. The high-energy shock wave of arc discharge may lead to vascular rupture, thrombus formation, and other high-risk complications. During the arc discharge process, the high temperature in the arc region may spread to surrounding tissues, causing unnecessary thermal damage. The shock wave of arc discharge has a strong one-time effect and is difficult to accurately control multiple times for cumulative effect treatment.

Cardiovascular diseases have long been one of the major threats to human health and life, and with the changes in modern lifestyles, the prevalence is increasing and affecting younger populations. Among these, diffuse vascular calcification is a major cause of severe cardiovascular diseases such as coronary heart disease and stroke. This condition is characterized by the proliferation of fibrous tissue in the vascular intima and the deposition of calcium, leading to thickened and hardened blood vessels and narrowed vascular lumen. Once the lumen becomes completely blocked, ischemia or even necrosis in the tissues or organs supplied by the blood vessels will occur. For severe diffuse calcification of the vascular wall, a surgical treatment method using a cutting balloon is generally adopted. However, this surgical treatment method can cause damage to the endothelial cells of the inner layer of the vessel during balloon expansion.

### SUMMARY OF THE PRESENT INVENTION

The technical problem to be solved by the present invention is to provide a balloon catheter based on liquid corona discharge and its control method, which can improve the safety, ease of operation, reusability, and precision control of the balloon catheter.

To solve the aforementioned technical problem, the technical solution of the present invention is as follows:
A balloon catheter based on liquid corona discharge, comprising:
An inner tube;
A balloon closedly arranged around the inner tube;
A corona generator located in the containment space between the inner tube and the balloon; electrolyte liquid is stored in the containment space between the inner tube and the balloon;
A power pulse generator electrically connected to the corona generator;

The corona generator is located in the electrolyte liquid in the containment space between the balloon and the inner tube; the corona generator receives pulse electrical signals sent by the power pulse generator, and under the influence of the pulse electrical signal, the electrolyte liquid undergoes a corona reaction. The electrolyte liquid generates molecular ionization, and the vapor bubbles produced by molecular ionization compress the electrolyte liquid, increasing the pressure within the balloon and driving the balloon to expand radially along the inner tube.

Optionally, the balloon catheter based on liquid corona discharge further comprises: an outer tube that is closedly arranged around the inner tube and sealedly connected to the balloon, the cross-sectional diameter of the balloon is larger than the cross-sectional diameter of the outer tube, the containment space between the inner tube and the outer tube is connected and stores electrolyte liquid.

Optionally, the corona generator is electrically connected to the power pulse generator through conducting wires, the conducting wires are either co-extruded integrally with the inner tube, or fixed coaxially in the containment space between the inner tube and the balloon, with the conducting wires being wrapped by an insulating material on the outside.

Optionally, the corona generator comprises at least two electrodes, the at least two electrodes are arranged at a predetermined distance from each other, and both electrodes are fixedly connected to the inner tube, the at least two electrodes receive pulse electrical signals sent by the power pulse generator and, under the influence of the pulse electrical signals, the electrolyte liquid undergoes a corona reaction.

Optionally, the corona generator comprises a first electrode and a second electrode, the first electrode and the second electrode are spaced apart by a first predetermined distance; wherein the first electrode is connected to the positive terminal of the power pulse generator, while the second electrode is connected to the negative terminal of the power pulse generator, the first electrode and the second electrode receive pulse electrical signals sent by the power pulse generator, and form an electric field between the electrodes, under the influence of the pulse electrical signals, the electrolyte liquid undergoes a corona reaction.

Optionally, the corona generator comprises a third electrode, a fourth electrode and a fifth electrode, the third electrode, the fourth electrode and the fifth electrode are arranged sequentially along the inner tube, the adjacent electrodes are spaced apart by a second predetermined distance; wherein the third electrode and the fifth electrode are connected to the positive terminal of the power pulse generator, while the fourth electrode is connected to the negative terminal of the power pulse generator, the third, fourth, and fifth electrodes receive pulse electrical signals sent by the power pulse generator, and form an electric field between the electrodes, under the influence of the pulse electrical signals, the electrolyte liquid undergoes a corona reaction.

Optionally, the corona generator comprises a sixth electrode, a seventh electrode, an eighth electrode, a ninth electrode and a tenth electrode, the sixth electrode, the seventh electrode, the eighth electrode, the ninth electrode and the tenth electrode are arranged sequentially, the adjacent electrodes are spaced apart by a third predetermined distance, wherein the sixth electrode, the eighth electrode and the tenth electrode are connected to the positive terminal of the power pulse generator, while the seventh electrode and the ninth electrode are connected to the negative terminal of the power pulse generator, the sixth, seventh, eighth, ninth and tenth electrodes receive pulse electrical signals sent by the power pulse generator, and form an electric field between the electrodes, under the influence of the pulse electrical signals, the electrolyte liquid undergoes a corona reaction.

Optionally, the power pulse generator comprises:
A diode;
A capacitor electrically connected to the diode;
An insulated-gate bipolar transistor electrically connected to the diode and the capacitor;
A first resistor electrically connected to the insulated-gate bipolar transistor;
A second resistor electrically connected to the first resistor;
A relay group electrically connected to the insulated-gate bipolar transistor and the first resistor.

Optionally, the balloon is a non-compliant or a low-compliance balloon.

In one embodiment of the present invention, a control method for the balloon catheter based on liquid corona discharge is further provided, which comprises: applying the balloon catheter based on liquid corona discharge as described in the above solution, where the corona generator is located in the containment space between the inner tube and the balloon, the containment space between the inner tube and the balloon stores electrolyte liquid, the corona generator is located in the electrolyte liquid in the containment space between the balloon and the inner tube; the method includes:
Receiving control commands;
According to the control commands, receiving pulse electrical signals sent by the power pulse generator with a preset pulse width, and under the influence of the pulse electrical signals, the electrolyte liquid undergoes a corona reaction, generating molecular ionization, and the vapor bubbles produced by molecular ionization compress the electrolyte liquid, increasing the pressure within the balloon and driving the balloon to expand radially along the inner tube, transmitting the pressure to the target area; wherein the dilation size of the vapor bubbles is directly proportional to the pressure value within the balloon; the impulse generated by the outward dilation of the balloon is directly proportional to the duration of the vapor bubbles; the stress caused by the outward dilation of the balloon on the target area is directly proportional to the impulse.

The aforementioned solution of the present invention has the following technical effects:
The balloon catheter based on liquid corona discharge, as described in the embodiment, comprises: an inner tube; a balloon closedly arranged around the inner tube; a corona generator located in the containment space between the inner tube and the balloon; electrolyte liquid is stored in the containment space between the inner tube and the balloon; a power pulse generator electrically connected to the corona generator; the corona generator is located in the electrolyte liquid in the containment space between the balloon and the inner tube; the corona generator receives pulse electrical signals sent by the power pulse generator, and under the influence of the pulse electrical signal, the electrolyte liquid undergoes a corona reaction, the electrolyte liquid generates molecular ionization, and the vapor bubbles produced by molecular ionization compress the electrolyte liquid, increasing the pressure within the balloon and driving the balloon to expand radially along the inner tube, thereby improving the safety, ease of operation, reusability, and precision control of the balloon catheter.

### BRIEF DESCRIPTION OF THE DRAWINGS

FIG. 1 is a schematic view of the balloon catheter based on liquid corona discharge of the present invention.
FIG. 2 is a schematic view of the balloon catheter based on liquid corona discharge in an unfilled liquid state of the present invention
FIG. 3 is a schematic view of the balloon catheter based on liquid corona discharge in a filled liquid state of the present invention.
FIG. 4 is a schematic view of the vapor bubbles produced by corona discharge in the balloon catheter based on liquid corona discharge of the present invention.
FIG. 5 is a schematic view of the expansion of the vapor bubbles produced by corona discharge in the balloon catheter based on liquid corona discharge of the present invention.
FIG. 6 is a schematic view of the completion of treatment using the balloon catheter based on liquid corona discharge of the present invention.
FIG. 7 is a schematic view of the three-electrode setup of the balloon catheter based on liquid corona discharge of the present invention.
FIG. 8 is a schematic view of the vapor bubbles formed by the three-electrode setup in the balloon catheter based on liquid corona discharge of the present invention.
FIG. 9 is a schematic view of the expansion of the vapor bubbles formed by the three-electrode setup in the balloon catheter based on liquid corona discharge of the present invention.
FIG. 10 is a schematic view of the five-electrode setup of the balloon catheter based on liquid corona discharge of the present invention.
FIG. 11 is a schematic view of the vapor bubbles formed by the five-electrode setup in the balloon catheter based on liquid corona discharge of the present invention.
FIG. 12 is a schematic view of the balloon catheter based on liquid corona discharge of the present invention.
FIG. 13 is a circuit diagram of the power pulse generator for the balloon catheter based on liquid corona discharge of the present invention.
FIG. 14 is a graphical coordinate view of the relationship between vapor bubble expansion and current over time in the balloon catheter based on liquid corona discharge of the present invention.
FIG. 15 is a graphical coordinate view of the relationship between current and voltage over time in the balloon catheter based on liquid corona discharge of the present invention.

### Description of the reference signs in the drawings:

1- Inner tube, 2- outer shell, 21- outer tube, 22- balloon, 31- first electrode, 32- second electrode, 33- third electrode, 34- fourth electrode, 35- fifth electrode, 36-sixth electrode, 37- seventh electrode, 38- eighth electrode, 39- ninth electrode, 310-tenth electrode, 4- power pulse generator, 5- connector, 6- conducting wire, D- diode, C- capacitor, G- insulated-gate bipolar transistor, R1- first resistor, R2- second resistor, K- relay group.

### DETAILED DESCRIPTION OF THE PREFERRED EMBODIMENT

The following description is used to disclose the exemplary embodiments of the present disclosure in more detail with reference to the drawings. Although the exemplary embodiments of the present disclosure are illustrated in the drawings, it should be understood that the present disclosure can be implemented in various forms and should not be limited to the embodiments set forth herein. In contrary, the provided embodiments are for better understanding and comprehending the full scope of the present disclosure for those skilled in the art.

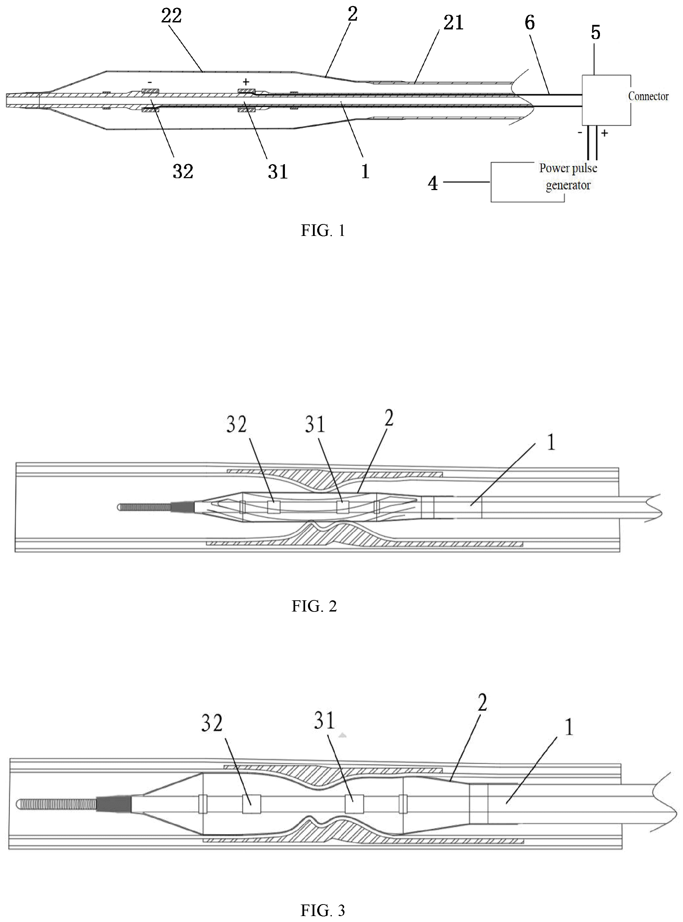

As shown in FIG. 1, an embodiment of the present invention provides a balloon catheter based on liquid corona discharge, which comprises:
An inner tube 1;
A balloon 22 closedly arranged around the inner tube 1;
A corona generator located in the containment space between the inner tube 1 and the balloon 22; electrolyte liquid is stored in the containment space between the inner tube 1 and the balloon 22;
A power pulse generator 4 electrically connected to the corona generator;

The corona generator is located in the electrolyte liquid in the containment space between the balloon 22 and the inner tube 1; the corona generator receives pulse electrical signals sent by the power pulse generator 4, and under the influence of the pulse electrical signals, the electrolyte liquid undergoes a corona reaction, the electrolyte liquid generates molecular ionization, and the vapor bubbles produced by molecular ionization compress the electrolyte liquid, increasing the pressure within the balloon 22 and driving the balloon 22 to expand radially along the inner tube 1.

In this embodiment, as shown in FIG. 1, the corona generator is placed in the electrolyte liquid. By receiving pulse electrical signals sent by the power pulse generator 4, the corona generator induces a corona reaction in the electrolyte liquid, which causes molecular ionization. The ionized molecules produce high-temperature plasma that vaporizes the surrounding liquid, producing vapor bubbles. As the ionization reaction continues, the vapor bubbles expand, compressing the surrounding liquid environment. Since the liquid cannot be compressed, the internal pressure of the balloon 22 instantly increases, driving the balloon 22 to expand radially. When the ionization reaction ends, the balloon returns to its original state. The duration of the ionization reaction is determined by the pulse width of the preset signal.

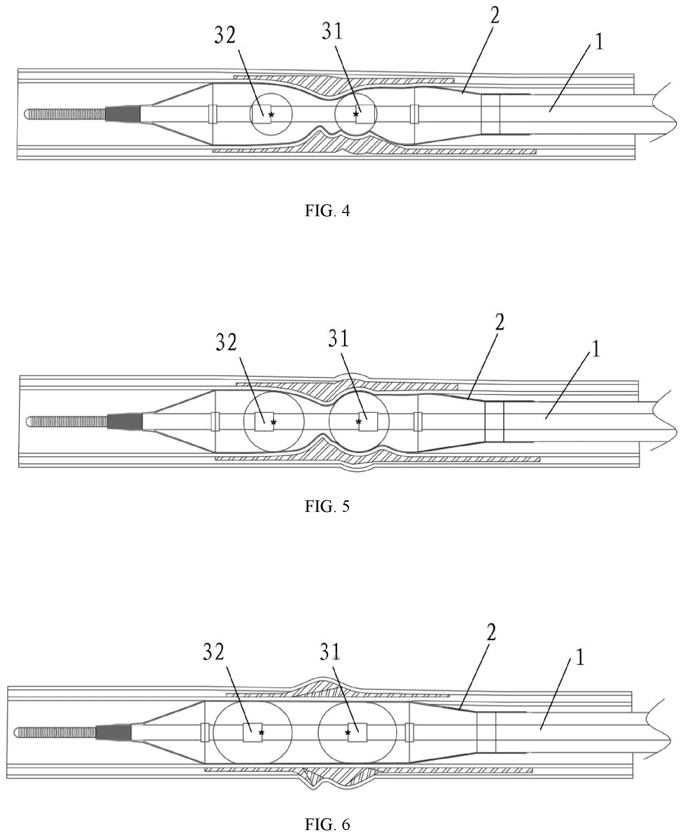

The balloon catheter based on liquid corona discharge can be used for treating vascular stenosis and calcified lesions. During use, first, under imaging guidance, the balloon catheter is introduced to the target site, i.e., the target blood vessel segment, using a guide wire. As shown in FIG. 2, when the balloon 22 is in an unfilled state with electrolyte liquid, the balloon catheter can easily pass through the stenotic and calcified regions. After the balloon catheter reaches the target position, electrolyte liquid is filled into the balloon 22 through the catheter system to a preset pressure of 2 atmospheres, as shown in FIG. 3, causing the balloon to attach to the vessel wall and ensuring full contact with the lesion area. A high-voltage pulse is applied, as shown in FIG. 4, causing the corona generator to produce corona discharge without reaching breakdown discharge intensity. The corona discharge phenomenon produces vapor bubbles in the electrolyte liquid, which compress the electrolyte liquid, increasing the pressure within the balloon 22, as shown in FIG. 5, thereby driving the balloon 22 to expand radially along the inner tube 1, and applying pressure to the target area, breaking the calcified material and expanding the vessel lumen, as shown in FIG. 6. Depending on the degree of the lesion and treatment needs, multiple corona discharges can be performed to ensure thorough removal of the lesion area. After each discharge, the balloon 22 can be slightly deflated, and the catheter position can be adjusted to cover the entire lesion area. After treatment is completed, the balloon 22 is deflated, and the catheter is withdrawn from the body. Postoperative imaging is used to ensure the smoothness of the blood vessel, thereby achieving a successful treatment. This solution enables the effective breakdown of the calcium within the calcified lesion in the blood vessel through the controlled and instantaneous radial expansion of a balloon, preparing the blood vessel for subsequent treatment. This greatly improves the efficacy, safety, ease of operation, reusability, and precision control during calcified vessel interventional treatment.

In one optional embodiment of the present invention, the balloon catheter based on liquid corona discharge further comprises:

An outer tube 21 that is closedly arranged around the inner tube 1 and sealedly connected to the balloon 22, the cross-sectional diameter of the balloon 22 is larger than the cross-sectional diameter of the outer tube 21, the containment space between the inner tube 1 and the outer tube 22 is connected and stores electrolyte liquid.

In one optional embodiment of the present invention, the balloon catheter based on liquid corona discharge further comprises:
A connector 5, the corona generator and the power pulse generator 4 are electrically connected through the connector 5, and the corona generator receives pulse electrical signals sent by the power pulse generator 4 through the connector 5.

In this embodiment, the connector 5 can control whether the corona generator is powered and can set voltage levels, making the operation of the balloon catheter more convenient.

In one optional embodiment of the present invention, the corona generator is electrically connected to the power pulse generator 4 through conducting wires 6, the conducting wires 6 are either co-extruded integrally with the inner tube 1, or fixed coaxially in the containment space between the inner tube 1 and the balloon 22, with the conducting wires 6 being wrapped by an insulating material on the outside.

In this embodiment, the corona generator uses the conducting wires 6 to electrically connect to the power pulse generator 4, or it can be electrically connected to the power pulse generator 4 through the connector 5. During manufacturing, the conducting wires 6 can be co-extruded integrally with the inner tube 1, which improves the overall integrity of the inner tube and extends the lifespan and stability of the balloon catheter. Alternatively, the conducting wires 6 can be fixed axially along the inner tube 1 and positioned in the containment space between the inner tube 1 and the balloon 22, the conducting wires 6 are wrapped by an insulating material on the outside to prevent voltage leakage.

In this embodiment, the corona generator and the power pulse generator 4 are directly and electrically connected by using the conducting wires 6, receiving pulse electrical signals sent by the power pulse generator 4.

In one optional embodiment of the present invention, the conducting wires 6 and at least two electrodes are fixedly connected by laser welding, resistance welding, ultrasonic welding, argon arc welding, plasma welding, or physical crimping.

In this embodiment, the fixed connection between the wires 6 and at least two electrodes can be achieved by the following methods: preferably laser welding under appropriate laser power conditions, preferably resistance welding under suitable current conditions, preferably ultrasonic welding under appropriate frequency conditions, preferably argon arc welding under suitable current conditions, preferably plasma welding under suitable current conditions, or physical crimping.

As shown in FIG. 1, in one optional embodiment of the present invention, the corona generator comprises at least two electrodes, the at least two electrodes are arranged at a predetermined distance from each other, and both electrodes are fixedly connected to the inner tube 1, the at least two electrodes receive pulse electrical signals sent by the power pulse generator 4, and under the influence of the pulse electrical signals, the electrolyte liquid undergoes a corona reaction.

In this embodiment, as shown in FIG. 1, the corona generator comprises at least two electrodes, the two electrodes are fixed to the inner tube 1 and spaced at a predetermined distance that prevents breakdown discharge between the electrodes, the electrodes are arranged on the inner tube 1, and at least two electrodes are required to trigger the corona discharge phenomenon;
The electrode pair is made of high-temperature-resistant materials, such as 304 stainless steel, 316 stainless steel, or tungsten alloys; under the condition of excellent conductivity, the material can withstand the thermal erosion of the high-temperature plasma generated during the corona discharge process; the electrode pair needs to be arranged at a predetermined distance to prevent electric arc between the electrodes, which would result in breakdown discharge, electrolyte liquid needs to be filled in the distance to form a conductive path environment. Under certain parameters, the longer the distance between the electrode pairs, the greater the resistance of the conductive path, and the smaller the current formed in the path.

In one optional embodiment of the present invention, the corona generator comprises a first electrode 31 and a second electrode 32, the first electrode 31 and the second electrode 32 are spaced apart by a first predetermined distance; wherein the first electrode 31 is connected to the positive terminal of the power pulse generator 4, while the second electrode 32 is connected to the negative terminal of the power pulse generator 4, the first electrode 31 and the second electrode 32 receive pulse electrical signals sent by the power pulse generator 4, and form an electric field between the electrodes, under the influence of the pulse electrical signals, the electrolyte liquid undergoes a corona reaction.

In this embodiment, as shown in FIG. 1, when the corona generator comprises two electrodes, one electrode is connected to the positive terminal of the power pulse generator 4, while the other is connected to the negative terminal, ensuring the vapor bubbles produced by the corona discharge are evenly distributed; the first electrode 31 and the second electrode 32 are spaced apart by a first predetermined distance, the first predetermined distance is the distance at which only molecular ionization occurs between the electrodes without breakdown discharge; as shown in FIG. 4, vapor bubbles are produced around the end of the relative side of the electrodes 31 and 32, as the electrodes are continuously powered, the vapor bubbles continue to grow, as shown in FIGS. 5 and 6, until they drive the balloon 22 to expand, thereby completing the treatment.

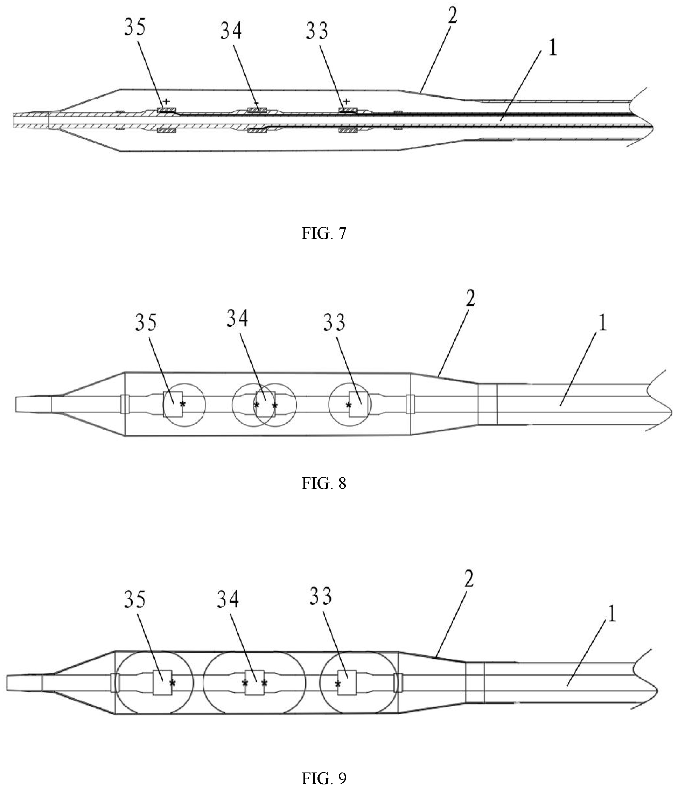

In one optional embodiment of the present invention, as shown in FIG. 7, the corona generator comprises a third electrode 33, a fourth electrode 34 and a fifth electrode 35, the third electrode 33, the fourth electrode 34 and the fifth electrode 35 are arranged sequentially along the inner tube 1, the adjacent electrodes are spaced apart by a second predetermined distance; wherein the third electrode 33 and the fifth electrode 35 are connected to the positive terminal of the power pulse generator 4, while the fourth electrode 34 is connected to the negative terminal of the power pulse generator 4, the third 33, fourth 34, and fifth 35 electrodes receive pulse electrical signals sent by the power pulse generator 4, and form an electric field between the electrodes, under the influence of the pulse electrical signals, the electrolyte liquid undergoes a corona reaction.

In this embodiment, as shown in FIG. 7, when the corona generator comprises three electrodes, the electrode located in the middle is connected to the negative terminal of the power pulse generator 4, while the two electrodes located on both sides are connected to the positive terminal, ensuring the vapor bubbles produced by the corona discharge are evenly distributed; the three electrodes are arranged sequentially along the inner tube 1, the adjacent electrodes are spaced apart by a second predetermined distance, the second predetermined distance is the preferred optimal distance at which only molecular ionization occurs between the electrodes without breakdown discharge; as shown in FIG. 8, vapor bubbles are produced around the end of the relative side of the electrodes 33 and 34, similarly, vapor bubbles are also produced around the end of the relative side of the electrodes 35 and 34, as the electrodes are continuously powered, the vapor bubbles continue to grow, as shown in FIGS. 9, until they drive the balloon 22 to expand, thereby completing the treatment.

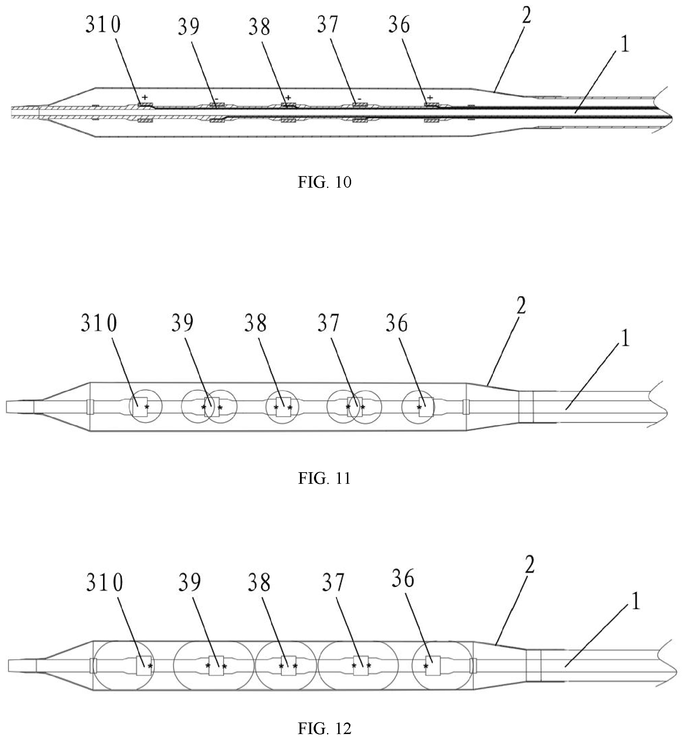

As shown in FIG. 10, in one optional embodiment of the present invention, the corona generator comprises: a sixth electrode 36, a seventh electrode 37, an eighth electrode 38, a ninth electrode 39 and a tenth electrode 310, the sixth electrode 36, the seventh electrode 37, the eighth electrode 38, the ninth electrode 39 and the tenth electrode 310 are arranged sequentially, the adjacent electrodes are spaced apart by a third predetermined distance, wherein the sixth electrode 36, the eighth electrode 38 and the tenth electrode 310 are connected to the positive terminal of the power pulse generator 4, while the seventh electrode 37 and the ninth electrode 39 are connected to the negative terminal of the power pulse generator 4, the sixth 36, seventh 37, eighth 38, ninth 39 and tenth 310 electrodes receive pulse electrical signals sent by the power pulse generator 4, and form an electric field between the electrodes, under the influence of the pulse electrical signals, the electrolyte liquid undergoes a corona reaction.

In this embodiment, as shown in FIG. 10, when the corona generator comprises five electrodes, the electrodes located at the both ends and the middle are connected to the positive terminal of the power pulse generator 4, and the remaining two electrodes are connected to the negative terminal, the positive electrodes and the negative electrodes are arranged in a staggered manner to ensure the vapor bubbles produced by the corona discharge are evenly distributed; the five electrodes are arranged sequentially along the inner tube 1 and are spaced apart by a third predetermined distance, the third predetermined distance is the preferred optimal distance at which only molecular ionization occurs between the electrodes without breakdown discharge;
As shown in FIG. 11, vapor bubbles are produced around the end of the relative side of the adjacent electrodes, as the electrodes are continuously powered, the vapor bubbles continue to grow, as shown in FIGS. 12, until they drive the balloon 22 to expand, thereby completing the treatment.

In one optional embodiment of the present invention, the electrolyte liquid used is 0.9% sodium chloride saline solution.

In this embodiment, the electrolyte liquid is 0.9% sodium chloride saline solution, which ensures the normal occurrence of the corona discharge phenomenon.

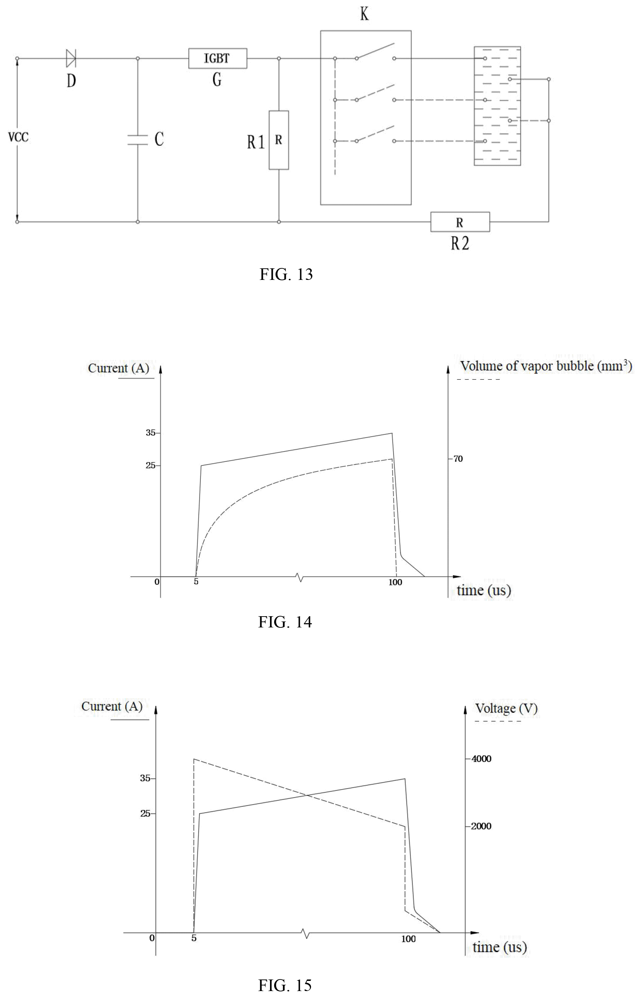

As shown in FIG. 13, in one optional embodiment of the present invention, the power pulse generator 4 comprises:
A diode D;
A capacitor C electrically connected to the diode D;
An insulated-gate bipolar transistor G electrically connected to the diode D and the capacitor C;
A first resistor R1 electrically connected to the insulated-gate bipolar transistor G;
A second resistor R2 electrically connected to the first resistor R1;
A relay group K electrically connected to the insulated-gate bipolar transistor G and the first resistor R1.

In this embodiment, as shown in FIG. 13, the power pulse generator 4 can set both the voltage value (1000-8000V) and the pulse width of the released charge (1-200µs) in the circuit, and can connect to at least one pair of electrodes. The power pulse generator 4 uses a built-in battery, where the high-voltage charge is stored in the capacitor C through the step-up of a high-voltage module; the diode D acts as a rectifier, after the current flows through the diode D, the negative voltage is filtered to form a unidirectional pulse current, the unidirectional pulse current flows through the capacitor C, charging the capacitor C, and the capacitor C stores high-voltage charge. The first resistor R1 and the second resistor R2 are respectively the working loads at both ends of the relay group K, ensuring the safety of the circuit; when the high-voltage charges need to be released to the electrodes, the circuit is opened and closed by the coordination of the IGBT (insulated-gate bipolar transistor G) and the relay group K, when one of the paths of the relay group K is closed, the IGBT conducts, releasing the high-voltage charge stored in the capacitor C through the connected circuit to form a high-voltage pulse; when multiple pairs of electrodes need to generate arcs simultaneously or sequentially, multiple energy storage capacitors and multiple relays are coordinated to manage the circuit's opening and closing; the current threshold value in the circuit can be set with feedback, and the pulse width of the high-voltage charge release can be achieved by coordinating with the delay function of the power supply.

In one optional embodiment of the present invention, the balloon 22 is a non-compliant balloon or a low-compliance balloon.

In this embodiment, the balloon 22 is a non-compliant balloon or a low-compliance balloon, a non-compliant balloon refers to a balloon that, once the diameter of the balloon 22 reaches a certain value, it will maintain that value regardless of changes in external pressure; a low-compliance balloon refers to a balloon that, once its diameter reaches a certain value, any further increase in size is relatively small, therefore, the material of the balloon 22 is selected from high-strength, high-flexibility polymers.

The present invention proposes a solution where the balloon is arranged with two electrodes with a relatively large distance between them, and a high-voltage pulses are applied between the electrodes, using the vapor bubbles produced by the corona discharge phenomenon to expand and compress the liquid inside the balloon. Due to the incompressibility of the liquid, the internal pressure of the balloon increases due to the compression of the liquid molecules. This phenomenon occurs within 0.1-0.2 milliseconds, and the impact on the vascular endothelium is limited. The briefly pressurized balloon expands instantly, compressing the calcified stenosis inside the blood vessel, causing the calcification to breakdown, the continuous generation of the vapor bubbles gradually dilate the calcified stenosis within the blood vessel, preparing the blood vessel for subsequent treatment.

When the corona discharge occurs in the liquid, at least two electrodes are required, and a high voltage is applied between them. The high voltage causes the liquid medium near the electrode surface to ionize, forming plasma. The characteristic of corona discharge is that the electric field strength around the electrodes is sufficient to cause molecular ionization in the liquid, but the distance between the electrodes is large enough to prevent breakdown discharge.

Under the influence of the high voltage, the electric field strength near the electrode surface increases significantly. A needle-shaped or wire-shaped electrode design can concentrate the electric field, making the electric field strength at the electrode tip reach its maximum. Under the influence of the strong electric field, the liquid molecules are ionized, producing high-energy electrons and ions. These high-energy electrons collide with liquid molecules, further ionizing more molecules and forming a plasma region. The temperature of the plasma region rises rapidly, typically reaching thousands of degrees Celsius. Due to the high temperature of the plasma region, the liquid near the electrodes heated up rapidly. When the local temperature exceeds the boiling point of the liquid, the liquid begins to vaporize, forming vapor bubbles. This process is extremely rapid, typically completing within milliseconds or even shorter time. The initial vapor bubbles, due to the high temperature and the high pressure, expand and grow rapidly. The volume of the vapor bubbles continues to increase with changes in temperature and pressure. During this process, the temperature and pressure of the surrounding liquid will also affect the growth rate and final size of the vapor bubbles.

In one embodiment of the present invention, a control method for the balloon catheter based on liquid corona discharge is further provided, which applies to the balloon catheter based on liquid corona discharge as described in the above embodiment, where the corona generator is located in the containment space between the inner tube 1 and the balloon 22, electrolyte liquid is stored in the containment space between the inner tube 1 and the balloon 22, the corona generator is located in the electrolyte liquid in the containment space between the balloon 22 and the inner tube 1; the method includes:

### Receiving control commands;

According to the control commands, receiving pulse electrical signals sent by the power pulse generator 4 with a preset pulse width, and under the influence of the pulse electrical signals, the electrolyte liquid undergoes a corona reaction, generating molecular ionization, and the vapor bubbles produced by molecular ionization compress the electrolyte liquid, increasing the pressure within the balloon 22 and driving the balloon 22 to expand radially along the inner tube 1, transmitting the pressure to the target area; wherein the dilation size of the vapor bubbles is directly proportional to the pressure value within the balloon; the impulse generated by the outward dilation of the balloon is directly proportional to the duration of the vapor bubbles; the stress caused by the outward dilation of the balloon on the target area is directly proportional to the impulse.

In this embodiment, the expansion of vapor bubbles is affected by the resistance of the liquid medium and surface tension. During the expansion process, the internal pressure of the vapor bubbles gradually decreases, when the pressure inside the vapor bubbles balances with the pressure of the surrounding liquid, the expansion of the vapor bubbles stops. The behavior and characteristics of the vapor bubbles produced by corona discharge are influenced by various factors, including voltage, pulse width, electrode shape, and liquid properties. The applied voltage and pulse width directly affect the intensity and duration of corona discharge. Higher voltage and longer pulse widths typically produce larger vapor bubbles. The shape and spacing of the electrodes affect the distribution and intensity of the local electric field. Needle-shaped or wire-shaped electrodes can concentrate the electric field, forming a stronger local electric field, thereby promoting the formation and expansion of the vapor bubbles. The thermophysical properties of the liquid, such as boiling point, thermal conductivity, and viscosity, will also affect the formation and growth process of the vapor bubbles. The behavior of the vapor bubbles may differ significantly in different liquid media.

Due to the corona discharge phenomenon, high-temperature charges ionize the electrolyte between the electrodes, forming high-temperature plasma, the vaporized electrolyte forms vapor bubbles that continuously expand and compress the surrounding solution, causing the internal pressure of the balloon to increase instantly and expand outward. The size of the vapor bubbles is directly proportional to the pressure value within the balloon, and the greater the maximum volume of the vapor bubbles relative to the fixed volume of the balloon, the higher the pressure. The impulse generated by the outward dilation of the balloon is directly proportional to the duration of the vapor bubbles; the stress caused by the outward dilation of the balloon on the target area is directly proportional to the impulse; as shown in FIG. 14, the size of the vapor bubbles is directly proportional to the current value of the corona discharge path, and the duration of the vapor bubbles is directly proportional to the pulse width of the corona discharge path; as shown in FIG. 15, the current of the corona discharge path is directly proportional to the voltage.

The balloon catheter based on liquid corona discharge described in the present invention provides the following advantages compared to the prior art:
1. In the aspect of precision control, the expansion process of vapor bubbles produced by corona discharge can be precisely controlled, by adjusting the voltage and discharge time, the size, expansion speed, and duration of vapor bubbles can be accurately regulated.
2. In the aspect of progressive treatment, the expansion of vapor bubbles through corona discharge can be a gradual process, allowing for the gradual dilation of calcified regions, reducing the sudden impact on the vascular wall, thereby lowering the risk of tissue damage.
3. In the aspect of risk control, the expansion force of vapor bubbles generated by corona discharge is relatively mild, and the mechanical impact on surrounding tissues is minimal, reducing the risk of intraoperative and postoperative complications.
4. In the aspect of thermal damage control, the local heating and vaporization effects of corona discharge are concentrated between the electrodes, preventing a large-scale thermal effect and minimizing thermal damage to surrounding tissues.
5. In the aspect of repeatability and controllability, the vapor bubble expansion process from corona discharge can be repeated multiple times, and the desired dilation effect can be achieved through gradual accumulation. This repeatability and controllability contribute to safer and more effective treatment.

For high-risk patients and cases requiring multiple treatments, the vapor bubble expansion from corona discharge method may be a more ideal option.

The above description of the preferred embodiments of the present invention should be understood as exemplary rather than limiting. For those skilled in the art, numerous modifications and improvements can be made without departing from the basic principles of the invention. Such modifications and improvements are also considered within the scope of protection of the present invention.

## Claims

1. A balloon catheter based on liquid corona discharge, **characterized in that**, comprising:
an inner tube (1);
a balloon (22) closedly arranged around said inner tube (1);
a corona generator located in the containment space between said inner tube (1) and said balloon (22); electrolyte liquid is stored in the containment space between said inner tube (1) and said balloon (22);
a power pulse generator (4) electrically connected to said corona generator;
said corona generator is located in said electrolyte liquid in the containment space between said balloon (22) and said inner tube (1); said corona generator receives pulse electrical signals sent by said power pulse generator (4), and under the influence of said pulse electrical signals, said electrolyte liquid undergoes a corona reaction, said electrolyte liquid generates molecular ionization, and the vapor bubbles produced by molecular ionization compress said electrolyte liquid, increasing the pressure within said balloon (22) and driving said balloon (22) to expand radially along said inner tube (1).

2. The balloon catheter based on liquid corona discharge, as recited in claim 1, **characterized in that**, further comprises: an outer tube (21) that is closedly arranged around said inner tube (1) and sealedly connected to said balloon (22), the cross-sectional diameter of said balloon (22) is larger than the cross-sectional diameter of said outer tube (21), the containment space between said inner tube (1) and said outer tube (22) is connected and stores electrolyte liquid.

3. The balloon catheter based on liquid corona discharge, as recited in claim 1, **characterized in that**, said corona generator is electrically connected to said power pulse generator (4) through conducting wires (6), said conducting wires (6) are either co-extruded integrally with said inner tube (1), or fixed coaxially in the containment space between said inner tube (1) and said balloon (22), with said conducting wires (6) being wrapped by an insulating material on the outside.

4. The balloon catheter based on liquid corona discharge, as recited in claim 1, **characterized in that**, said corona generator comprises at least two electrodes, said at least two electrodes are arranged at a predetermined distance from each other, and both electrodes are fixedly connected to said inner tube (1), said at least two electrodes receive pulse electrical signals sent by said power pulse generator (4), and under the influence of said pulse electrical signals, said electrolyte liquid undergoes a corona reaction.

5. The balloon catheter based on liquid corona discharge, as recited in claim 4, **characterized in that**, said corona generator comprises:
a first electrode (31) and a second electrode (32), said first electrode (31) and said second electrode (32) are spaced apart by a first predetermined distance; wherein said first electrode (31) is connected to the positive terminal of said power pulse generator (4), while said second electrode (32) is connected to the negative terminal of said power pulse generator (4), said first electrode (31) and said second electrode (32) receive pulse electrical signals sent by said power pulse generator (4), and form an electric field between said electrodes, under the influence of said pulse electrical signals, said electrolyte liquid undergoes a corona reaction.

6. The balloon catheter based on liquid corona discharge, as recited in claim 4, **characterized in that**, said corona generator comprises:
a third electrode (33), a fourth electrode (34) and a fifth electrode (35), said third electrode (33), said fourth electrode (34) and said fifth electrode (35) are arranged sequentially along said inner tube (1), the adjacent electrodes are spaced apart by a second predetermined distance; wherein said third electrode (33) and said fifth electrode (35) are connected to the positive terminal of said power pulse generator (4), while said fourth electrode (34) is connected to the negative terminal of the power pulse generator (4), said third electrode (33), said fourth electrode (34), and said fifth electrode (35) receive pulse electrical signals sent by said power pulse generator (4), and form an electric field between said electrodes, under the influence of said pulse electrical signals, said electrolyte liquid undergoes a corona reaction.

7. The balloon catheter based on liquid corona discharge, as recited in claim 4, **characterized in that**, said corona generator comprises:
a sixth electrode (36), a seventh electrode (37), an eighth electrode (38), a ninth electrode (39) and a tenth electrode (310), said sixth electrode (36), said seventh electrode (37), said eighth electrode (38), said ninth electrode (39) and said tenth electrode (310) are arranged sequentially, the adjacent electrodes are spaced apart by a third predetermined distance, wherein said sixth electrode (36), said eighth electrode (38) and said tenth electrode (310) are connected to the positive terminal of said power pulse generator (4), while said seventh electrode (37) and said ninth electrode (39) are connected to the negative terminal of said power pulse generator (4), said sixth electrode (36), said seventh electrode (37), said eighth electrode (38), said ninth electrode (39) and said tenth electrode (310) receive pulse electrical signals sent by said power pulse generator (4), and form an electric field between said electrodes, under the influence of said pulse electrical signals, said electrolyte liquid undergoes a corona reaction.

8. The balloon catheter based on liquid corona discharge, as recited in claim 1, **characterized in that**, said power pulse generator (4), comprising:
a diode (D);
a capacitor (C) electrically connected to said diode (D);
an insulated-gate bipolar transistor (G) electrically connected to said diode D and said capacitor (C);
a first resistor (R1) electrically connected to said insulated-gate bipolar transistor (G);
a second resistor (R2) electrically connected to said first resistor (R1);
a relay group (K) electrically connected to said insulated-gate bipolar transistor (G) and said first resistor (R1).

9. The balloon catheter based on liquid corona discharge, as recited in any one of claims 1-8, **characterized in that**, said balloon (22) is a non-compliant balloon or a low-compliance balloon.

10. A control method for the balloon catheter based on liquid corona discharge, **characterized in that**, applying to the balloon catheter based on liquid corona discharge as described in claim 1, where said corona generator is located in the containment space between said inner tube (1) and said balloon (22); said electrolyte liquid is stored in the containment space between said inner tube (1) and said balloon (22), said corona generator is located in said electrolyte liquid in the containment space between said balloon (22) and said inner tube (1); said method includes:
Receiving control commands;
According to the control commands, receiving pulse electrical signals sent by said power pulse generator (4) with a preset pulse width, and under the influence of said pulse electrical signals, said electrolyte liquid undergoes a corona reaction, generating molecular ionization, and vapor bubbles produced by molecular ionization compress said electrolyte liquid, increasing the pressure within said balloon (22) and driving said balloon (22) to expand radially along said inner tube (1), transmitting the pressure to the target area; wherein the dilation size of the vapor bubbles is directly proportional to the pressure value within the balloon; the impulse generated by the outward dilation of the balloon is directly proportional to the duration of the vapor bubbles; the stress caused by the outward dilation of the balloon on the target area is directly proportional to the impulse.
